(19) Europäisches Patentamt
European Patent Office
Office européen des brevets

(11) Publication number: 0 164 569 B1

(12) EUROPEAN PATENT SPECIFICATION

(45) Date of publication of patent specification: 31.10.90

(51) Int. Cl.$^5$: A 61 K 9/22

(21) Application number: 85105589.7

(22) Date of filing: 07.05.85

# (54) A medicine-releasing agent.

(30) Priority: 11.05.84 JP 92749/84

(43) Date of publication of application:
18.12.85 Bulletin 85/51

(45) Publication of the grant of the patent:
31.10.90 Bulletin 90/44

(84) Designated Contracting States:
BE CH DE FR GB IT LI NL SE

(56) References cited:
EP-A-0 130 935
FR-A-2 205 306
US-A-3 867 520
US-A-4 351 337

DIE PHARMAZIE, vol. 34, no. 7, 1979, pages 415-417, VEB VERLAG VOLLE UND GESUNDHET, BERLIN, DD; A.H.FGHANEM et al. "The effect of a second laminated gelatin film on the release rate of sulfadiazine from gelatin-gel matrix"

(73) Proprietor: KOKEN CO. LTD.
5-18 Shimoochiai 3-chome
Shinjuku-ku Tokyo (JP)

(72) Inventor: Kuroyanagi, Yoshimitsu
1429-30, Hatsusawa-cho
Hachiogi-shi Tokyo (JP)
Inventor: Miyata, Teruo
6-29, 314, Shimo-ochiai 3-chome
Shinjuku-ku Tokyo (JP)
Inventor: Seno, Manabu
17-26-604, Takada 2-chome
Toshima-ku Tokyo (JP)
Inventor: Kubota, Tetsuaki
32-6, Hachimanyama 3-chome
Setagaya-ku Tokyo (JP)

(74) Representative: Kraus, Walter, Dr. et al
Patentanwälte Kraus, Weisert & Partner
Thomas-Wimmer-Ring 15
D-8000 München 22 (DE)

EP 0 164 569 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to a triple-layered medicine-releasing agent made of medicine complex, prepared from medicine mixture solution composed of medicine and natural or synthetic polypeptide, and poly-alpha-amino acid, provided with hydrophilic and hydrophobic radicals. More particularly, this invention pertains to an agent that can release a compounded medicine in living tissues extending over a few months for example, which is not only excellent in the assimilation and the affinity with cellular tissues but widely improved in the durability of the medicinal efficacy.

For example, in order to increase the medicinal efficacy against cancer and lessen the undesirable side effects as much as possible, it is considered most effective from the chemotherapeutic point of view to embed a certain kind of carrier impregnated with a carcinostatic substance near an affected part by conducting an operation. For this reason, recently carriers aimed at such effect have been increasingly used as they release impregnated medicines slowly. At present, natural polymers such as gelatin, agar and cellulose derivatives or synthetic polymers such as vinyl acetate, polymethyl methacrylate and polyvinyl alcohol are used as carriers. However, natural polymers liberate medicine too easily, and are therefore inferior in the durability of medicinal efficacy; contrary to this, synthetic polymers have problems in the affinity with living tissues and the ease of assimilation in a living body.

US—A—4 351 337 describes an implantable drug delivery device comprising a matrix formed of a poly-α-amino acid component having one or more drug and/or diagnostic agents physically contained therein.

The present inventors made an extensive study to eliminate the above drawbacks and found that a poly-α-amino acid having hydrophilic radicals and hydrophobic radicals together shows an appropriate permeability for medicine; and the rate of the permeability depends on the content of hydrophilic radicals. They have thus finally accomplished the present invention.

Accordingly it is the object of the present invention to provide a medicine-releasing agent not only superior in the assimilation for and affinity with cellular tissues in a living body but improved in the durability of the medicinal efficacy.

According to the present invention this object is defined by an agent as defined above which is characterized in that said hydrophilic radical is selected from glutamate, N-hydroxyalkyl-L-glutamate, and dihydroxyethylaminopropyl-L-glutamate radicals, and that said hydrophobic radical is a gamma-benzyl-L-glutamate radical, the molar ratio of said hydrophilic radicals to said hydrophobic radicals being in the range from 0.1:0.9 to 0.3:0.7.

Figs. 1 and 3 are a schematic diagram showing a production process of the medicine-releasing agent according to the present invention. Figs. 2 and 4 are a graph showing the progress that the medicine-releasing agent produced in accordance with Example 1 and Example 2 releases a compounded medicine with the passage of time.

In the present invention, a poly-α-amino acid that has good affinity with living cellular tissues and an appropriate permeability for medicine, which is employed to encapsulate a medicinal compound, is a copolymer that has hydrophilic radicals and hydrophobic radicals together as shown by the following structural formulae, of which molecular weight is 100,000—200,000.

(1) Copoly(γ-benzyl-L-glutamyl-L-glutamic acid)

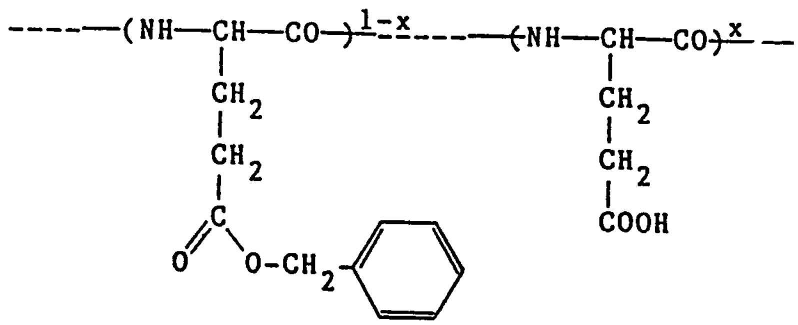

(2) Copoly(γ-benzyl-L-glutamyl-$N^5$-hydroxyalkyl-L-glutamine)

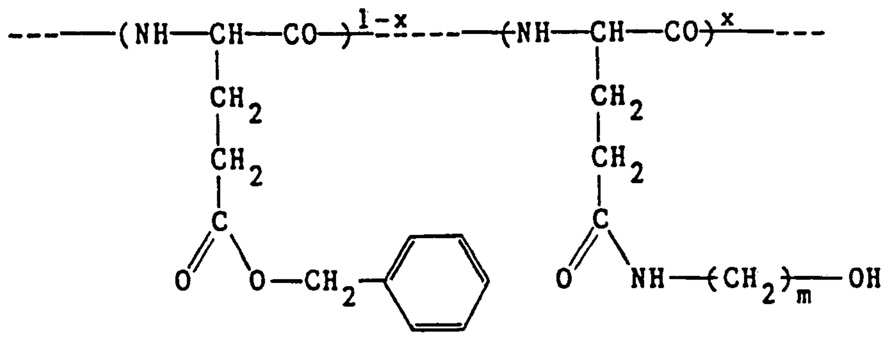

(m=2-4)

(3) Copoly(γ-benzyl-L-glutamyl-$N^5$-dihydroxyethylaminopropyl-L-glutamine)

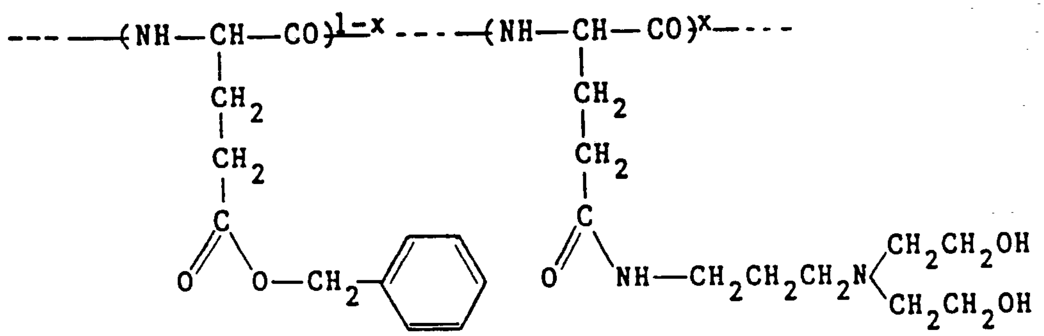

wherein x stands for the molar number of hydrophilic radicals. The optimal value of x is different from medicine to medicine according to their molecular weight as they are allowed to pass through the copolymer membrane, but it commonly ranges from 0.1 to 0.3. In connection with this the present inventors have already published a synthesizing process for copolymers of this kind in Journal of Polymer Science, Polymer Chemistry Edition, *21*, 1289—1303, (1983).

In this invention, generally speaking, a natural/synthetic polypeptide is used for the sake of carrying a medicine and forming a compound therewith. However, considering good affinity with living cellular tissues and easiness of assimilation in a living body, poly-α-amino acid homologues, atero collagen and succinylated collagen are desirable, and yet copolymers represented by the above formulae 1, 2 and 3 are preferable among the poly-α-amino acid homologues. Besides, a water-soluble poly-α-amino acid can also be employed so long as a water-soluble medicine is employed.

(4) Poly($N^5$-hydroxyalkyl-L-glutamine)

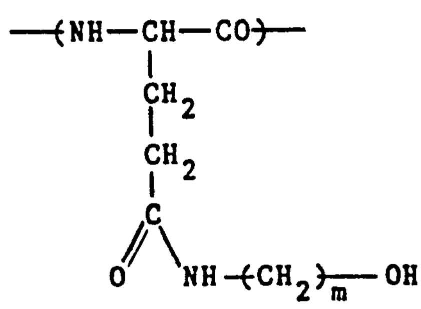

(m=2-4)

It would rather be said that if only a medicine can form a compound together with the polypeptide mentioned above, there is no other restriction in particular for what kind of medicine to be used. For example, carcinostatic substances such as 5-fluorouracil (5-FU), adriamycin, cispratin, and pepleomycin, and steroid hormones such as pledonisolone and estradiol, can be enumerated here only for illustration.

In the meanwhile, as a result of having examined the relation between the value of x and the permeability for medicine by the employment of a copoly(γ-benzyl-L-glutamyl-$N^5$-dihydroxyethylaminopropyl-L-glutamine) membrane of thickness 20 μm, whose chemical structure is shown by the formula (3), it becomes clear that even a high molecular weight carcinostatic substance such as pepleomycin easily passes through the membrane in a few hours when x is 0.6. Therefore, the copolymer was considered improper for a permeable membrane to encapsulate a medicine in according to this invention.

However, when x is 0.3, the coplymer membrane allows a low molecular weight medicine to pass through in a few hours and allows a high molecular weight medicine to pass through in a few weeks. From this, the copolymer proved beneficial and promising as a membrane for encapsulating a high molecular weight medicine such as adriamycin and pepleomycin.

Moreover, it was proved that when x is 0.2, the copolymer membrane can liberate a low molecular weight medicine extending over a few weeks; therefore, it proved suitable as a membrane for medicines with relatively low molecular weight such as 5-fluorouracil.

Like this, as for copoly(γ-benzyl-L-glutamyl-L-glutamic acid) whose chemical structure is shown by the formula (1) or copoly(γ-benzyl-L-glutamyl-$N^5$-hydroxypropyl-L-glutamyl) whose chemical structure is shown by the formula (2) (provided m is equal to 3 in this case), there is also an optimal value of x in terms of the membrane permeability which is closely related with the molecular weight of medicines.

The medicine-releasing agent of this invention, characterized by having a three-layered structure, can for example be prepared as follows. That is, a medicinal compound which is to form a core of the medicine-releasing agent is prepared by casting a solution mixture on a flat plate and evaporating a solvent therefrom, where the solution mixture is made up by mixing a poly-α-amino acid solution with a medicine. Separately, a poly-α-amino acid membrane having an appropriate permeability for medicine which is to form a coat of the medicinal compound is prepared from its solution similarly to the above. Next, the medicinal compound is wrapped up in the poly-α-amino acid membrane; at this moment, a solvent, such as chloroform or dimethyl formamide, which can swell or dissolve the poly-α-amino acid is applied on the membrane a little to help it glue before pressing and then dried.

To show an example of its shape, the medicine-releasing agent of this invention is constructed of a medicinal compound of diameter 10—20 μm and thickness 50—100 μm and an outer membrane of thickness 10—50 μm; consequently, the two elements form into a three-layered structure.

A manufacturing process and a cross section of the medicine-releasing agent are schematically shown in Fig. 1.

The present invention will be understood more readily with reference to the following examples, which are intended to illustrate the invention.

### Example 1

At first, 0.5 g of predonisolon is dissolved in 10 ml of a dimethyl formamide solution containing copoly(γ-benzyl-L-glutamyl-$N^5$-hydroxypropyl-L-glutamine) whose chemical structure is shown by the formula (2), of which x is 0.3 so as to make the polymer consistency equal to 5 g/l. A sheet of a medicinal compound is made on top of a Teflon® plate by casting the solution and evaporating the solvent thereon. The medicinal compound having an area of 1 $cm^2$ is cut off from the sheet. Separately, a polymer membrane of thickness 10 μm is made on top of another Teflon® plate from a dimethyl formamide solution containing copoly(γ-benzyl-L-glutamyl-$N^5$-hydroxypropyl-L-glutamine) by casting the solution and evaporating the solvent. A medicine-releasing agent consisting of three layers is manufactured by wrapping up the medicinal compound in the polymer membrane.

As a result of studying the amount of released predonisolone, it was proved that 50 percent of the medicine dissolves out in isotonic sodium chloride solution for about a month at a constant rate, as shown in Fig. 2.

Two other medicine-releasing agents composed of a core comprising predonisolone and the poly-α-amino acid of the formula (1)/(2) and an outer membrane of copoly(γ-benzyl-L-glutamyl-$N^5$-dihydroxyethylaminopropyl-L-glutamine) individually gave a result similar to the above.

### Example 2

Two grams of succinylated collagen, which has been fully purified by a freeze-drying process, is dissolved in 80 ml of distilled water. A solution prepared by dissolving 1 g of pepleomycin in 20 ml of distilled water is added to the above solution with vigorous stirring by means of a homogenizer while the whole solution is being maintained below 20°C. There forms a cream-like viscous white solution. The solution is spread on the bottom of a square stainless steel container having a side of 10 cm so as to make a sponge-like medicine-polymer compound by freeze-drying. A pillar of diameter about 10 mm is cut off from the sponge and shaped into a disc by applying a pressure of $5.89.10^7$Pa (600 kg/$cm^2$). Separately, a membrane of thickness 30 μm is prepared from a dimethyl formamide solution which contains copoly(γ-benzyl-L-glutamyl-$N^5$-dihydroxyethylaminopropyl-L-glutamine) by casting the solution flat on a Teflon® plate and evaporating the solvent.

The medicinal compound thus prepared in the form of a disc is wrapped in the polymer membrane to make up a medicine-releasing agent. This process is shown in Fig. 3. The medicine-releasing agent is

soaked in isotonic sodium chloride solution at 37°C to scrutinize how much medicine is released with the passage of time. The result is given in Fig. 4, from which it is proved that about 50 percent of pepleomycin dissolves out of the polymer membrane for about a month at a constant rate.

Example 3

Except that copoly(γ-benzyl-L-glutamyl-$N^5$-dihydroxyethylaminopropyl-L-glutamine), used in Example 2, is replaced by poly($N^5$-hydroxyethyl-L-glutamine), all the processes and the materials are left unchanged as in Example 2 to prepare a medicine-releasing agent.

Example 4

The medicine-releasing agent prepared in Example 2 is used to examine how much medicine is released therefrom in a living body. For the sake of this, after having been sterilized with ethylene oxide, the medicine-releasing agent is embedded in a subcutaneous tissue of the femur of mice. Mice are killed one after another 5 days, 10 days, 15 days, 20 days, 25 days and 30 days after the embedding of the medicine-releasing agent in order to determine the amount of pepleomycin released in the tissue around the medicine-releasing agent.

As a result of each determination, which is made on one gram of the removed tissue, it becomes obvious that a certain amount of pepleomycin is liberated from time to time from the medicine-releasing agent at a constant rate extending over a month.

In this way, it becomes apparent that the assimilation of the medicine-releasing agent is made very well in a living body since poly-α-amino acid, atero collagen or succinylated collagen is used as a carrier for medicine; what is more, the releasing rate of medicines is put under good control supported by an appropriate permeability of the membrane that dependes on the amount of hydrophilic radicals contained in the poly-α-amino acid. Specifically, when the amount of hydrophilic radicals is in the range 10—20 percent, in other words, when the value of x is in the range of 0.1—0.2, the membrane is fit for a medicine with low molecular weight. Furthermore, in terms of a medicine with high molecular weight, a membrane whose value of x is in the range 0.2—0.3 is used appropriately. As apparent from this, according to the present invention, the releasing rate of medicine can be varied at will by changing a molar ratio of hydrophilic radicals to hydrophobic ones in a poly-α-amino acid to be used as an outer membrane. Besides, all the poly-α-amino acid of this invention intended for the use are so excellent that they would never be encapsulated by the surrounding cells with the result that they are eventually absorbed in them, together with a carrier for medicine a few months after embedded.

**Claim**

A triple-layered medicine-releasing agent made of medicine complex, prepared from medicine mixture solution composed of medicine and natural or synthetic polypeptide, and poly-alpha-amino acid, provided with hydrophilic and hydrophobic radicals, characterized in that said hydrophilic radical is selected from glutamate, N-hydroxyalkyl-L-glutamate, and dihydroxyethylaminopropyl-L-glutamate radicals, and that said hydrophobic radical is a gamma-benzyl-L-glutamate radical, the molar ratio of said hydrophilic radicals to said hydrophobic radicals being in the range from 0.1:0.9 to 0.3:0.7.

**Patentanspruch**

Dreischichtiges Arzneiwirkstoff freisetzendes Mittel aus einem Arzneiwirkstoffkomplex, der aus einer Arzneiwirkstoffgemischlösung aus Arzneiwirkstoff und einem natürlichen oder synthetischen Polypeptid hergestellt wurde und aus einer Poly-alpha-aminosäure, die hydrophile und hydrophobe Reste enthält, dadurch gekennzeichnet, daß der hydrophile Rest aus Glutamat-N-Hydroxy-L-Glutamat und Dihydroxyethylaminopropyl-L-Glutamat-Resten ausgwehält ist, und daß der hydrophobe Rest ein gamma-Benzyl-L-Glutamat-Rest ist, wobei das molare Verhältnis des hydrophilen Rests zu dem hydrophoben Rest im Bereich von 0,1:0,9 bis 0,3:0,7 liegt.

**Revendication**

Agent à trois couches libérant un médicament fait d'un complexe du médicament, préparé à partir d'une solution du mélange de médicament composé d'un médicament et de polypeptide naturel ou synthétique, et d'acide poly-alpha-aminé, pourvu de radicaux hydrophiles et hydrophobes, caractérisé en ce que ce radical hydrophile est choisi parmi les radicaux glutamate, N-hydroxyalkyl-L-glutamate et dihydroxyéthylaminopropyl-L-glutamate et que ce radical hydrophobe est un radical gamma-benzyl-L-glutamate, le rapport molaire de ces radicaux hydrophiles à ces radicaux hydrophobes étant dans le gamme de 0,1:0,9 à 0,3:0,7.

FIG. 1

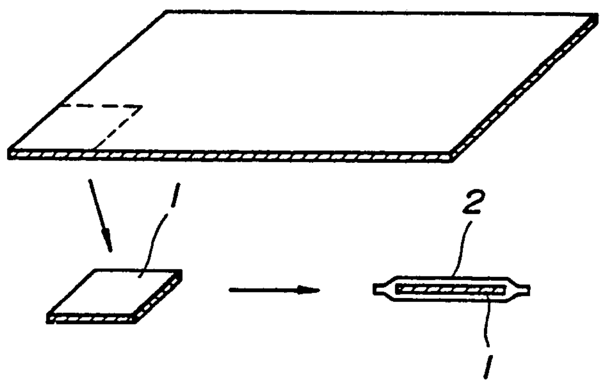
1
2
1

FIG. 2

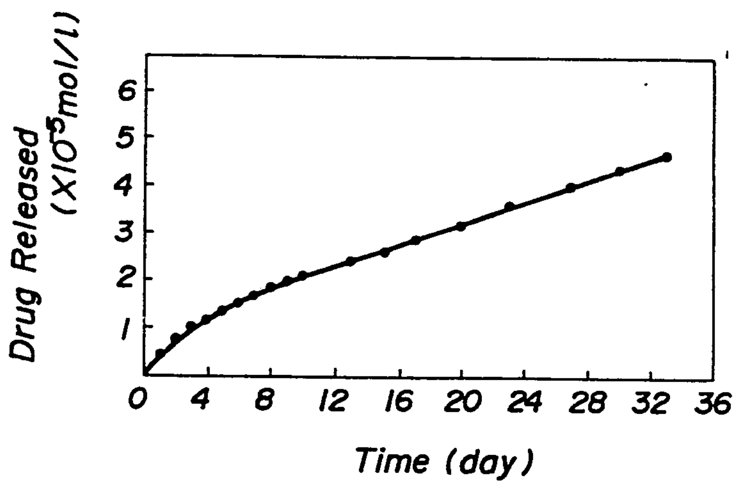
Drug Released
(X10⁻⁵mol/L)
6
5
4
3
2
1
0
4
8
12
16
20
24
28
32
36
Time (day)

FIG. 3

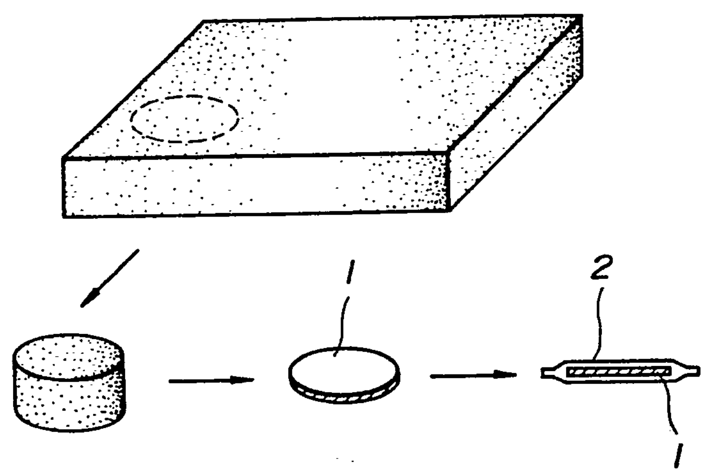
1
2
1

FIG. 4

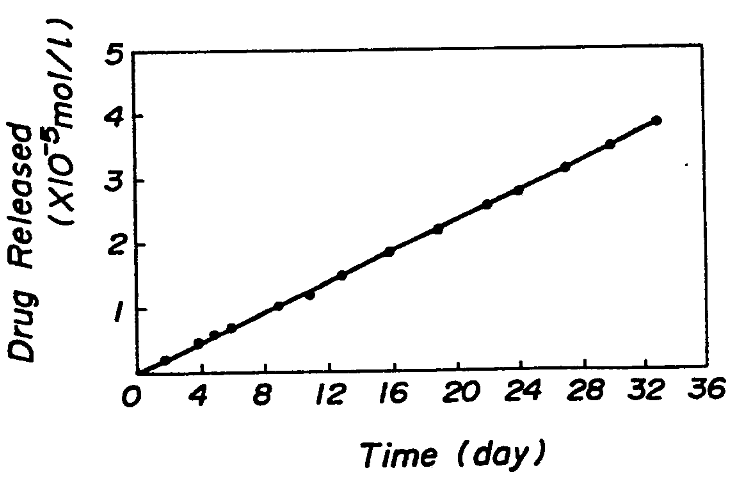
Drug Released
(X10$^{-5}$mol/l)
5
4
3
2
1
0
4
8
12
16
20
24
28
32
36
Time (day)